# FASCICULE DE BREVET EUROPEEN

(11) **EP 0 898 579 B1**
(45) Date de publication et mention de la délivrance du brevet: **23.02.2000**
(21) Numéro de dépôt: 97920776.8
(22) Date de dépôt: 17.04.1997
(51) Int. Cl.: C07J 41/00, A61K 31/57, C07J 9/00, C07J 7/00, C07J 31/00

(54) **DERIVES DE PREGNANE SANS SUBSTITUENT EN POSITION 17-ALPHA-, LEUR APPLICATION A TITRE DE MEDICAMENT, LEUR PROCEDE DE PREPARATION, LES INTERMEDIAIRES DE CE PROCEDE ET LES COMPOSITIONS LES CONTENANT**
17-ALPHA-UNSUBSTITUIERTE PREGNAN DERIVATE, IHRE VERWENDUNG ALS MEDIKAMENTE, EIN VERFAHREN ZU IHRER HERSTELLUNG UND ZWISCHENPRODUKTE DIESES VERFAHRENS, UND PHARMAZEUTISCHE PRÄPARATE DAVON
PREGNANE DERIVATIVES WITH NO ALPHA-17 SUBSTITUTENT, THEIR MEDICINAL USE, MANUFACTURING METHOD AND ITS INTERMEDIARIES AND RELATED COMPOUNDS

(30) Priorité: 18.04.1996 FR 9604845
(43) Date de publication de la demande: 03.03.1999
(73) Titulaire: HOECHST MARION ROUSSEL, 92800 Puteaux (FR)
(72) Inventeur: BHATNAGAR, Neerja, F-91600 Savigny-sur-Orge (FR); CLAUSSNER, André, F-93250 Villemomble (FR); MARCHANDEAU, Christian, F-77410 Annet-sur-Marne (FR); RESCHE RIGON, Michèle, F-75011 Paris (FR); TEUTSCH, Jean-Georges, F-93500 Pantin (FR)
(74) Mandataire: Vieillefosse, Jean-Claude
(86) Numéro de dépôt international: FR9700693
(87) Numéro de publication internationale: WO9739018

(56) Documents cités:
- EP-A- 0 320 253
- WO-A-87/01706
- FR-M- 7 605
- US-A- 2 837 544
- US-A- 5 223 493
- US-A- 5 420 120
- JOURNAL OF MEDICINAL CHEMISTRY, vol. 29, no. 11, 1986, WASHINGTON US, pages 2125-2136, XP002017806 T STOUCH ET AL: "Computer Aided Studies of the Structure Activity Relationships between the Structure of Some Steroids and Their Antiinflammatory Activity"
- JOURNAL OF MEDICINAL CHEMISTRY, vol. 26, no. 3, 1983, WASHINGTON US, pages 318-328, XP002017807 N BODOR ET AL: "Structure Activity Relationships in the Antiinflammatory Steroids: A Pattern-Recognition Approach"
- CHEMICAL AND PHARMACEUTICAL BULLETIN, vol. 38, no. 3, 1990, TOKYO JP, pages 692-697, XP002017808 MITSUKUCHI M ET AL: "Studies on topical antiinflammatory agents. V. 17-(Alkylthio)- and methoxyalkanoates of corticosteroids"
- BIOCHEMISTRY, vol. 18, no. 22, 1979, EASTON, PA US, pages 4915-4922, XP002017809 SIMONS S S, JR. ET AL: "Fluorescent chemoaffinity labeling. Potential application of a new affinity labeling technique to glucocorticoid receptors"
- DATABASE WPI Section Ch, Week 8904 Derwent Publications Ltd., London, GB; Class B01, AN 89-029179 XP002017811 & JP 63 303 994 A (TAISHO PHARMACEUT KK) , 12 Décembre 1988
- DATABASE WPI Section Ch, Week 8608 Derwent Publications Ltd., London, GB; Class B01, AN 86-051952 XP002017812 & JP 61 005 093 A (MITSUBISHI CHEM IND KK) , 10 Janvier 1986 & PATENT ABSTRACTS OF JAPAN vol. 010, no. 144 (C-349), 27 Mai 1986 & JP 61 005093 A (MITSUBISHI KASEI KOGYO KK), 10 Janvier 1986,
- JOURNAL OF MEDICINAL CHEMISTRY, vol. 33, no. 4, Avril 1990, WASHINGTON US, pages 1145-1151, XP002017810 E. JACOBSEN ET AL: "Novel 21-Aminosteroids That Inhibit Iron-Dependent Lipid Peroxidation and Protect against Central Nervous System Trauma"

## Description

La présente invention a pour objet des stéroides, leur application à titre de médicament, leur procédé de préparation et les intermédiaires de ce procédé, et les compositions pharmaceutiques les renfermant.

L'invention a pour objet les produits de formule générale (I) : 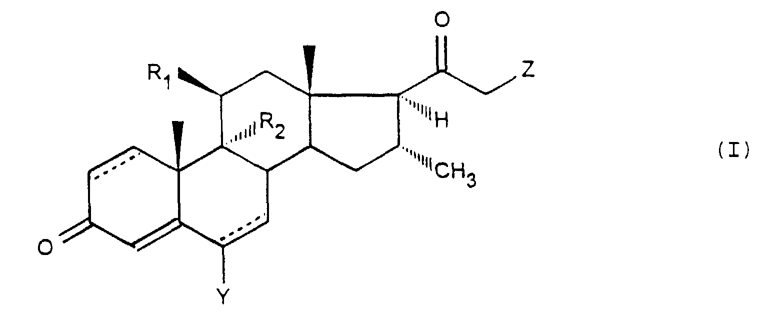 dans laquelle soit R₁ représente un atome d'halogène, un radical hydroxyle, un radical alkyloxy renfermant de 1 à 8 atomes de carbone, un radical acyloxy renfermant de 1 à 12 atomes de carbone, et R₂ représente un atome d'halogène ou un atome d'hydrogène, soit R₁ et R₂ forment ensemble une seconde liaison, Z est choisi parmi les groupements alkyloxy renfermant de 1 à 8 atomes de carbone, alkylthio renfermant de 1 à 8 atomes de carbone non substitué ou substitué, arylthio renfermant de 6 à 12 atomes de carbone non substitué ou substitué, halogène, cyano, mercapto, thiocyanato, CO₂A et CH₂CO₂A, A étant un atome d'hydrogène ou un groupement alkyle renfermant de 1 à 8 atomes de carbone, Y représente un atome d'hydrogène ou un méthyle, le trait en pointillé en position 1-2 ou 5-6 représentant éventuellement une seconde liaison, insi que leurs sels d'addition avec les acides ou les bases, étant entendu que le 9α-fluoro-11β-hydroxy-16α-méthyl-21-chloro-pregna-1,4-diène-3,20-dione est exclu et étant entendu que lorsque R₁ et R₂ forment ensemble une double liaison, Z n'est pas un atome d'halogène.

Par atome d'halogène, on entend les atomes de fluor, de brome, de chlore et d'iode.

Par radical alkyloxy et alkylthio renfermant de 1 à 8 atomes de carbone on entend de préférence les radicaux méthoxy, éthoxy, propoxy, butyloxy et les radicaux soufrés correspondants.

Par radical acyloxy renfermant de 1 à 12 atomes de carbone on entend de préférence les radicaux acétyloxy, propionyloxy, butyryloxy et benzoyloxy.

Par radical alkyle renfermant de 1 à 8 atomes de carbone, on entend les radicaux méthyle, éthyle, propyle, isopropyle, butyle, isobutyle, tert-butyle, n-pentyle, n-hexyle, 2-méthyl pentyle, 2,3-diméthyl butyle, n-heptyle, 2-méthylhexyle, 2,2-diméthylpentyle, 3,3-diméthylpentyle, 3-éthylpentyle, n-octyle, 2,2-diméthylhexyle, 3,3-diméthylhexyle, 3-méthyl-3-éthylpentyle. Il s'agit tout particulièrement des radicaux méthyle, éthyle, propyle, isopropyle, butyle, isobutyle et tert-butyle.

Par radical arylthio on entend de préférence le thiophényle.

Lorsque Z est un radical alkylthio ou arylthio substitué, il peut s'agir des substituants suivants : le fluor, le chlore, le brome, l'iode, un radical alkyle tel que méthyle, éthyle, propyle, isopropyle, butyle, isobutyle, tert-butyle, un radical alkoxy tel que méthoxy, éthoxy, propyloxy, isopropyloxy, butyloxy, un radical alkylthio tel que méthylthio, éthylthio, propylthio, isopropylthio, butylthio, un radical amino, un radical alkylamino tel que méthylamino ou éthylamino, un radical dialkylamino tel que diméthylamino, diéthylamino, méthyléthylamino, un radical hydroxyle éventuellement acylé, par exemple acétoxy, ou un radical de formule : -O-CO-(CH₂)ₙCO₂H dans lequel n= 2 à 5, un radical acyle renfermant de 2 à 8 atomes de carbone tel que acétyle, propionyle, butyryle, benzoyle, un radical carboxy libre, un radical carboxy estérifié tel que alkoxy carbonyle par exemple méthoxy carbonyle ou éthoxy carbonyle, un radical cyano, un radical trifluorométhyle, ou un radical phényle. Le terme alkyle renferme de 1 à 12 atomes de carbone.

Bien entendu, l'expression "éventuellement substitué" indique qu'un ou plusieurs substituants, identiques ou différents, peuvent être présents.

La substitution sur l'aryle peut s'effectuer en position ortho, méta ou para.

Lorsque Z est un radical alkylthio éventuellement substitué on entend de préférence le groupement S-CH₂-CH₂-A' dans lequel A' est un hydroxyle, un atome d'halogène ou un groupement acétyloxy.

Lorsque le cycle B est saturé, Y est de préférence en position α.

L'invention s'étend naturellement aux sels des composés de formule (I), lorsque ceux-ci comportent une fonction aminée, avec notamment les acides chlorhydrique, bromhydrique, nitrique, sulfurique, phosphorique, acétique, formique, propionique, benzoique, maléique, fumarique, succinique, tartrique, citrique, oxalique, glyoxylique, aspartique, alcane sulfoniques tels que les acides méthane et éthane sulfoniques, arylsulfoniques, tels que les acides benzène et paratoluène sulfoniques et arylcarboxyliques, et lorsque les composés de formule (I) comportent une fonction acide, aux sels des métaux alcalins, alcalino-terreux et d'ammonium éventuellement substitué.

L'invention a plus particulièrement pour objet les produits de formule (I) tels que définis précédemment dans lesquels R₁ est un radical hydroxyle et R₂ est un atome de fluor, ainsi que leurs sels d'addition avec les acides ou les bases.

Parmi les composés de l'invention, on peut citer de préférence les composés de formule (I) pour lesquels le cycle B est saturé et Y est un atome d'hydrogène, ainsi que leurs sels d'addition avec les acides ou les bases.

L'invention a plus particulièrement pour objet les produits de formule (I) tels que définis précédemment dans lesquels Z est un radical cyano ou un groupement alkylthio renfermant de 1 à 8 atomes de carbone.

Parmi les produits préférés de l'invention on peut citer les produits suivants :
- 9α-fluoro-11β-hydroxy-16α-méthyl-pregn-4-ène-3,20-dioxo-21-carbonitrile
- 3,20-dioxo-11β-hydroxy-16α-méthyl-21-nor cholane 1,4-diène-24-oate de méthyle
- 21-fluoro-11β-hydroxy-16α-méthyl-pregna-1,4-diène-3,20-dione
- 21-thio-(2-hydroxyéthylène)-9α,11β-dichloro-16α-méthylpregna-1,4-diene-3,20-dione
- 21-thio-(2-acétyloxyéthylène)-9α,11β-dichloro-16α-méthylpregna-1,4-diene-3,20-dione
- 9α,11β-dichloro-21-fluoro-16α-méthyl-pregna-1,4-diene-3,20-dione et tout particulièrement,
- 9α-fluoro-11β-hydroxy -16α-méthyl-pregna-1,4-diene-3,20-dioxo-21-carbonitrile,
- 9α-fluoro-11β-hydroxy-16α-méthyl-21-thiométhyl-pregna-1,4-diene-3,20-dione.

L'invention a également pour objet un procédé de préparation des produits de formule (I) caractérisé en ce que l'on soumet un produit de formule générale (II) : 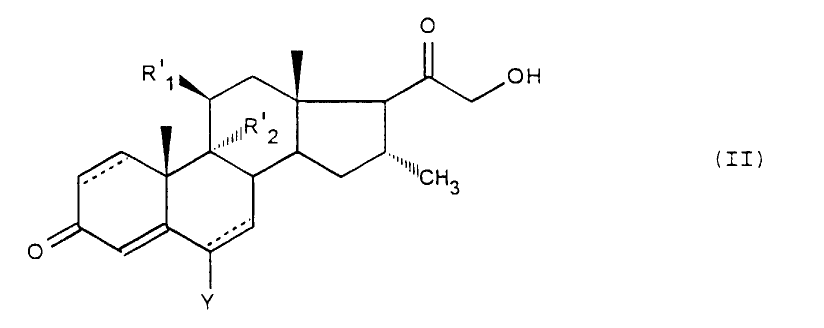 dans laquelle soit R'₁ est un hydroxyle et R'₂ est un halogène ou un hydrogène, soit R'₁ est un halogène et R'₂ est un halogène ou un hydrogène, soit R'₁ et R'₂ forment ensemble une seconde liaison et Y est tel que défini précédemment, à l'action d'un réactif d'activation de l'alcool de formule Hal-SO₂-B, Hal étant un atome de brome ou de chlore et B étant un radical alkyle renfermant de 1 à 6 atomes de carbone, non substitué ou substitué par 1 à 5 atomes d'halogène, ou un groupement phényle ou naphtyle non substitué ou substitué par 1 à 5 groupements alkyles, renfermant de 1 à 6 atomes de carbone, afin d'obtenir un composé de formule générale (III) : 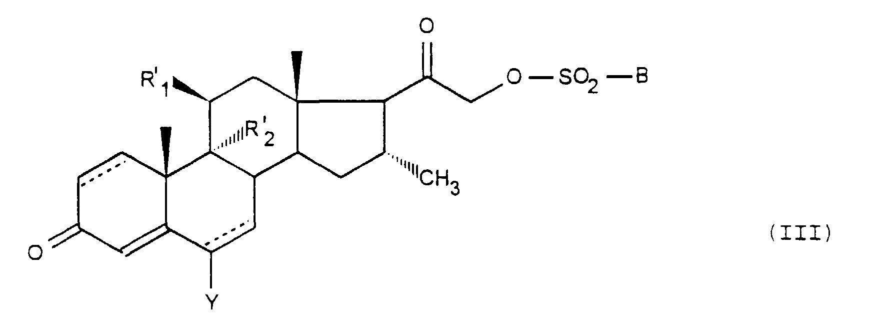 produit de formule (III) que l'on soumet, le cas échéant, à l'une ou plusieurs des réactions suivantes dans un ordre approprié afin d'obtenir un produit de formule générale (I) :
- action d'un réactif de chloration, de iodation, bromation ou fluoration,
- action d'un alkylthiol ou d'un arylthiol éventuellement substitué,
- action d'une thioamide ou d'une thiourée suivie d'une hydrolyse,
- action de KCN,
- hydrolyse du groupement cyano en milieu acide puis éventuellement estérification ou salification
- action successivement de CH₂(COOEt)₂, d'une réaction de saponification puis d'une réaction de décarboxylation suivie éventuellement d'une réaction d'estérification,
- action de KSCN ou NaSCN,
- action d'un alcool ou d'un alcoolate,
- réaction d'acylation en position 11,
- réaction d'alkylation en position 11,
- réduction de la double liaison en position 1-2,
- formation de la double liaison en position 1-2,
- réaction de déshydratation afin de former une double liaison en position 9-11,
- salification.

On entend de préférence par B les radicaux -CH₃,-CF₃, -Ph-Me.

Le mésylate, tosylate ou triflate de formule (III) se forme par action à froid, du chlorure de méthane sulfonyle, du chlorure de tosyle ou d'anhydride triflique sur l'alcool correspondant de formule (II) en présence d'une base telle que la pyridine.

La formation du dérivé 21-chloré à partir du mésylate de formule (III) correspondant s'effectue selon les méthodes connues de l'homme du métier, notamment par action de chlorure de lithium ou de potassium.

La formation du dérivé 21-bromé à partir du mésylate de formule (III) correspondant s'effectue selon les méthodes connues de l'homme du métier, notamment par action de bromure de lithium ou de potassium. On peut également obtenir directement les produits 21-bromés à partir des alcools correspondants par action d'acide bromhydrique ou de tribromure de phosphore.

La formation du dérivé 21-iodé à partir du mésylate de formule (III) correspondant s'effectue selon les méthodes connues de l'homme du métier, notamment par action de iodure de sodium ou de potassium.

La formation des dérivés 21-fluoro à partir des dérivés 21-chlorés, 21-bromés ou iodés correspondants s'effectue notamment par action de fluorure de potassium dans le glycol au reflux ou par l'emploi d'éther-couronne, par transfert de phase, ou par résine échangeuse d'ion. La formation du dérivé 21-fluoré à partir du mésylate de formule (III) correspondant s'effectue selon les méthodes connues de l'homme du métier, notamment par action de fluorure de potassium.

La formation des dérivés 21 alkylthio ou arylthio à partir du dérivé 21-chloré correspondant, s'effectue de préférence par action d'un alkylthiol ou arylthiol en présence d'une base telle que la triéthylamine dans le tétrahydrofuranne.

La formation du thiol correspondant s'effectue de préférence par une méthode indirecte telle que l'action d'un thioamide ou d'une thiourée suivie d'une hydrolyse.

La formation des dérivés 21-cyano s'effectue par action du cyanure de potassium en milieu éthanolique sur le dérivé 21-chloré, bromé ou iodé correspondant.

La réaction d'hydrolyse des groupements 21-cyano s'effectue de préférence en présence d'acide chlorhydrique ou d'acide sulfurique.

La formation des dérivés 21-thiocyanate s'effectue par action du thiocyanate de potassium ou de sodium en milieu éthanolique sur le dérivé 21-chloré correspondant.

L'action du malonate d'éthyle sur le dérivé 21-chloré afin d'obtenir le composé 21-CH(COOEt)₂ correspondant, s'effectue de préférence en présence d'une base forte telle que l'hydrure de sodium dans un solvant dipolaire aprotique tel que l'HMPT.

La réaction de saponification s'effectue selon les méthodes connues, par exemple en présence de soude en milieu éthanolique.

La réaction de décarboxylation s'effectue également selon les méthodes connues de l'homme du métier, notamment par voie thermique.

La réaction d'estérification s'effectue selon les méthodes connues de l'homme du métier, notamment par action de diazométhane. On peut également former préalablement un chlorure d'acide puis faire agir un alcool aliphatique.

La formation des dérivés 21-alkyloxy s'effectue de préférence par action d'un alcool aliphatique tel que CH₃OH ou nBuOH sur le dérivé 21-chloré dans un solvant dipolaire aprotique tel que le diméthylsulfoxyde en présence d'une base telle que la soude.

Les réactions de salification peuvent être effectuées dans des conditions usuelles. On opère par exemple en présence de soude éthanolique. On peut également utiliser un sel de sodium tel que le carbonate ou le carbonate acide de sodium ou de potassium.

La réaction de déshydratation des composés de formule générale (I), (II) ou (III) dans lesquels R₁ est un hydroxyle et R₂ est un atome d'hydrogène, afin d'obtenir les composés de formule (I) avec une double liaison en position 9-11 s'effectue selon les méthodes usuelles, parmi lesquelles on peut citer par exemple: l'action d'un chlorure de mésylate ou d'anhydride triflique suivie d'une réaction thermique.

Les produits de formule (I) possédant une seconde liaison en position 1-2 peuvent être réduits en produits de formule (I) saturés en position 1-2 par action d'une réaction d'hydrogénation selon les méthodes usuelles connues de l'homme du métier.

La formation d'une double liaison en position 1-2 peut s'effectuer selon les méthodes usuelles par voie enzymatique ou chimique, notamment par action de 2,3-dichloro-5,6-dicyanobenzoquinone (DDQ) dans le dioxane.

La réaction d'acylation de l'hydroxyle en position 11 s'effectue par action d'un acide carboxylique ou d'un anhydride carboxylique.

La réaction d'alkylation de l'hydroxyle en position Il s'effectue par exemple par action d'un iodure d'alkyle en présence d'une base telle que tertbutylate de potassium dans un solvant tel que le tétrahydrofuranne.

L'invention a également pour objet, à titre de produits industriels nouveaux, les produits de formule générale (III), à l'exception des composés suivants :
- 21-(méthanesulfonyloxy)-16α-méthylpregna-1,4,9(11)-trièn-3,20-dione
- 21-(méthanesulfonyloxy) 11β-hydroxy-16α-méthyl-pregna-1,4-dièn-3,20-dione et son dérivé 9α-Fluoro correspondant
- 21-(4-méthylphénylsulfonyloxy)-16α-méthyl-pregna-1,4,9(11)-trièn-3,20-dione.

Les produits de formule (II) utilisés comme produits de départ du procédé de préparation sont connus d'une façon générale. Il s'agit notamment de la désoxyméthasone (US3232839), du 9α,11β-dichloro 16α-méthyl 21-hydroxy pregna 1,4-diène-3,20-dione (Demande de Certificat d'addition n° 2381065), du 16α-méthyl 1,4-pregnadiène-11β,21-diol-3,20-dione (US3354186), du 6α,16α-diméthyl 1,4-pregnadiène-11β,21-diol-3,20-dione (US3232839) ou du 6α-fluoro,16α-méthyl 1,4-pregnadiène-11β,21-diol-3,20-dione (US3232839).

Parmi les molécules anti-inflammatoires et immunosuppressives actuellement disponibles, les glucocorticoïdes constituent une des plus puissantes classes thérapeutiques.

Leur utilisation est néanmoins limitée du fait de leurs nombreux effets secondaires, parmi lesquels on peut citer:
- effet frénateur de l'axe hypothalamo-hypophyso-surrénalien (HPA)
- intolérance au glucose, pouvant précipiter l'apparition d'un diabète
- fonte musculaire
- retard de la cicatrisation
- atrophie de la peau
- ostéoporose
- susceptibilité accrue aux infections
- troubles neurologiques
- hypercholestérolémie.

Chem Pharm Bull. vol 38(3) p. 692-7 (1990), EP-320,253, US-5,223,493, et US-5,420,120 décrivent des pregnan-3,20-diones anti-inflammatoires, substitués en position 17-alpha par un groupement hydroxy ou son ester.

Les effets des glucocorticoïdes sont médiés par des récepteurs nucléaires appartenant à la famille des récepteurs stéroïdiens. Ces récepteurs sont un facteur de transcription "ligand-inductible" pouvant moduler positivement ou négativement la transcription des gènes cibles. (Evans, R. M., 1988 Science, **240**, 889-895), (Green, S., Kumar, V., Theulaz, I., Wahli, W., and Chambon, P. 1988 EMBO J., **7**, 3037-3044), (Beato, M. 1989. Cell, 56, 335-344), (Jonat, C., Rahmansdorf, H.J., Park, K.K., Cato, A.C., Gebel, S., Ponta, H., Herrlich, P., 1990 Cell, **62**, 1189-1204), (Pfahl, M. 1993 Endocrine Reviews, **14**, 651-658).

L'utilisation des mutants des récepteurs des glucocorticoïdes a permis d'établir que des régions distinctes de ces récepteurs sont impliquées dans les fonctions de transactivation ou de transrépression, et de ce fait, que ces deux fonctions sont théoriquement dissociables (Heck, S., Kullmann, M., Gast, A., Ponta, H., Rahmansdorf, H.J., Herrlich, P., and Cato, A.C.B. 1994 EMBO J., **13**, 4087-4095).

L'obtention de ligands du récepteur glucocorticoide 5 agissant in vivo comme anti-inflammatoires, et dénués de fonction de transactivation, permettrait de développer des molécules mieux tolérées.

Les composés de formule générale (I) présentent d'intéressantes propriétés pharmacologiques :

### 1) Activité glucocorticoïde

La demanderesse a, en effet, mis en évidence une nouvelle classe de glucocorticoïdes. Différents modèles animaux (rats, souris) ont permis de mettre en évidence les très fortes propriétés anti-inflammatoires des produits de l'invention. Notamment ils possèdent une remarquable activité glucocorticoide par voie locale. (cf test de l'oedème de l'oreille induit par l'huile de croton chez la souris, activité in vivo équivalente ou supérieure à la prednisolone ou à la dexaméthasone).

### 2) Activité dissociée

Par ailleurs, les produits de l'invention agissent via le mécanisme d'action suivant : ces molécules permettent en effet de séparer les fonctions de transactivation et de transrépression du récepteur. Elles ont une activité dite "dissociée" sur la transcription des gènes cibles.

Les molécules selon l'invention ont été testées dans des modèles de cellules HELA transfectées avec le plasmide GRE-tk-CAT (transactivation), ou avec le plasmide pColl-CAT (transrépression). (Cf. TEST 1)

Ces molécules comme la DEXAMETHASONE sont capables d'inhiber la transcription du promoteur de la collagénase ; par contre, à la différence de la DEXAMETHASONE, elles n'activent pas ou très peu la transcription du promoteur GRE-tk.

Les produits décrits présentant des activités anti-inflammatoires, et immunosuppressives du même ordre que la PREDNISOLONE, les applications thérapeutiques restent les applications classiquement décrites pour les médicaments faits à partir de la PREDNISOLONE.

Ils peuvent par exemple être utilisés pour le traitement d'affections ou de maladies allergiques, dermatologiques, digestives, endocriniennes, hématologiques, infectieuses, néoplastiques, néphrologiques, neurologiques, ophtalmologiques, respiratoires ou rhumatologiques. Ils sont particulièrement intéressants dans les transplantations d'organes pour prévenir le rejet de greffes mais également pour le traitement des réactions inflammatoires locales comme par exemple, les oedèmes, les dermatoses, les prurits, les diverses formes d'eczéma, les érythèmes solaires, les tendinites ou les entorses. Ils sont également tout particulièrement intéressants pour le traitement des désordres inflammatoires ophtalmiques.

Leur activité dissociée rend les composés de l'invention particulièrement intéressants dans le traitement des maladies citées plus haut tout en diminuant certains effets secondaires.

L'invention a donc pour objet les produits de formule (I) ainsi que leurs sels d'addition avec les acides ou les bases pharmaceutiquement acceptables, à titre de médicaments.

L'invention a plus particulièrement pour objet les produits de formule (I), ainsi que leurs sels d'addition avec les acides ou les bases pharmaceutiquement acceptables, à titre de médicaments ayant une activité glucocorticoÏde.

L'invention a tout particulièrement pour objet les produits de formule (I), ainsi que leurs sels d'addition avec les acides ou les bases pharmaceutiquement acceptables, à titre de médicaments ayant une activité glucocorticoïde dissociée, cette dissociation permettant une diminution ou une disparition des effets secondaires.

Parmi les médicaments de l'invention on peut plus particulièrement citer :
- 9α-fluoro-11β-hydroxy-16α-méthyl-pregna-1,4-diene-3,20-dioxo-21-carbonitrile,
- 9α-fluoro-11β-hydroxy-16α-méthyl-3,20-dioxo-pregn-4-ène-21-carbonitrile,
- 9α-fluoro-11β-hydroxy-16α-méthyl-21-thiométhyl-pregna-1,4-diene-3,20-dione,
- 3,20-dioxo-11β-hydroxy-16α-méthyl-21-nor cholane 1,4-diène-24-oate de méthyle,
- 21-fluoro-11β-hydroxy-16α-méthyl-pregna-1,4-diène-3,20-dione.

Parmi les médicaments de l'invention on peut tout particulièrement citer :
- 9α-fluoro-11β-hydroxy-16α-méthyl-pregna-1,4-diene-3,20-dione-21-carbonitrile,
- 9α-fluoro-11β-hydroxy-16α-méthyl-21-thiométhyl-pregna-1,4-diene-3,20-dione.

La posologie utile varie en fonction de l'affection à traiter et de la voie d'administration. Elle peut varier par exemple de 1 à 1000 mg par jour chez l'adulte par voie orale.

L'invention s'étend aux compositions pharmaceutiques renfermant comme principe actif, au moins l'un des médicaments tel que défini ci-dessus.

Les composés de formule (I) sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparation injectables, de pommades, de crèmes, de gels, de microsphères, d'implants, de patchs, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie utile varie, notamment, en fonction du sujet à traiter et de l'affection en cause. Elle peut être comprise, par exemple, entre 1 et 4 application par jour d'une pommade renfermant de 0,1 % à 5 % de produit de l'exemple 1.

L'invention a tout particulièrement pour objet les compositions pharmaceutiques administrables par voie locale renfermant à titre de médicaments les composés suivants tels que décrits plus haut.

### Exemples

### Composés de formule (I)

### Préparation 1 : 9α-fluoro-11β-hydroxy-16α-méthyl-21-chloropregna-1,4-diene-3,20-dione (cf Mol. and Cell. Endocrinal (1981) 22 153-168)

Après dissolution de 6 g de désoxyméthasone dans 24 ml de pyridine, on ajoute 6 g de chlorure de lithium puis 6 ml de chlorure de l'acide méthane sulfonique en maintenant une température inférieure à 50°C puis on agite 2 heures à température ambiante. On verse dans l'eau, essore, lave et sèche le produit brut que l'on purifie par chromatographie sur silice en éluant avec le mélange chloroforme/acétate d'éthyle 2/8 pour obtenir 1,86 g de produit pur attendu.
F = 185°C
Infrarouge
- C=O: 1726 et 1707 cm⁻¹

### EXEMPLE 1 : 9α-fluoro-11β-hydroxy-16α-méthyl-21-thiométhylpregna-1,4-diene-3,20-dione

Après dissolution de 1,86 g du produit de la préparation 1 dans 20 ml de tétrahydrofuranne (THF) et 2 ml de triéthylamine (TEA), on fait barboter pendant 1 heure à 0°C du méthylthiol et agite 48 heures à température ambiante. On verse dans l'eau, essore, lave et sèche le produit brut que l'on purifie par recristallisation dans un mélange chlorure de méthylène (CH₂Cl₂) et d'éther isopropylique pour obtenir 1,477 g de produit pur attendu. F = 166°C
Infra-rouge (CHCl₃)
- OH: 3620 cm⁻¹ + associé
- C=O: 1715 (épaulement), 1694, 1666 cm⁻¹
- C=C: 1627, 1611 cm⁻¹

### EXEMPLE 2 : 9α-f1uoro-11β-hydroxy-16α-méthyl-pregna-1,4-diene-3,20-dione-21-carbonitrile

On mélange sous atmosphère inerte et au reflux pendant 1 h 30, 3,9 g du produit de la préparation 1, 39 ml d'éthanol, 1,080 g de cyanure de potassium (90 %) et 1,6 ml d'eau. Après refroidissement au bain de glace et ajustement au pH4 par addition d'acide acétique, on essore les sels minéraux et évapore sous pression réduite jusqu'à obtention de 4,7 g de produit brut que l'on purifie par chromatographie sur silice en éluant avec le mélange acétate d'éthyle/benzène 4/6. On obtient 2,7 g de produit pur attendu. F = 250°C
Infrarouge (CHCl₃)
- OH: 3615 cm⁻¹ + associé
- C=O: 1723, 1664 cm⁻¹
- Δ1-4: 1627, 1608cm⁻¹
- C≡N: 2260 cm⁻¹

### EXEMPLE 3 : 21-chloro-9α-fluoro-11β-hydroxy-16α-méthyl-pregn-4-ène-3,20-dione

On opère de manière équivalente à la préparation 1, mais à partir de 8 g de 16α-méthyl-9α-fluoro-pregn-4-ène-11β, 21-diol-3,20-dione (FR1315629). On obtient 4,95 g de produit pur attendu, après recristallisation dans le 2,2-diméthoxypropane. F = 196°C
Infrarouge (CHCl₃)
- OH: 3616 cm⁻¹ + associé
- C=O: 1725, 1706cm⁻¹
- Δ4: 1663, 1624cm⁻¹

### EXEMPLE 4 : 9α-fluoro-11β-hydroxy-16α-méthyl-3,20-dioxo-pregn-4-ène-21-carbonitrile

On opère comme à l'exemple 2 mais à partir de 3,450 g du produit de l'exemple 3. On obtient 2,36 g du produit pur attendu. F = 224°C
Infrarouge (CHCl₃)
- OH: 3610 cm⁻¹ + associé
- C=O: 1722 cm⁻¹
- Δ4: 1667, 1625cm⁻¹
- C≡N: 2260 cm⁻¹

### EXEMPLE 5 : 3,20-dioxo-11β-hydroxy-16α-méthyl-21-norcholane 1,4-diène-24-oate de méthyle

### Stade A : 21-chloro-16α-méthyl-pregna-1,4,9 (11)-triène-3-20-dione

A un mélange sous atmosphère inerte de 15 g de 16α-méthyl-21-hydroxy-pregna-1,4,9 (11)-triène-3-20-dione dans 40 ml de méthyl éthyl pyridine, on ajoute à 10°C, 8,8 ml de chlorure de méthane sulfonyle et agite à température ambiante pendant 5 heures. On verse dans un mélange glace + eau, acidifie par addition d'acide chlorydrique, filtre, lave et sèche. On obtient 16,2 g de produit brut attendu que l'on purifie par chromatographie en éluant avec le mélange benzène/acétate d'éthyle 8/2. On obtient 13,4 g de produit pur attendu. F = 154°C

### Stade B : 3,20-dioxo-23-éthoxycarbonyl-16α-méthyl-21-norchola-1,4,9(11)-triène 24-oate d'éthyle.

A un mélange sous atmosphère inerte de 1,860 g d'hydrure de sodium à 50 % dans 50 ml d'HMPT et 6 ml de malonate d'éthyle, on ajoute 13,9 g du produit chloré obtenu au stade précédent dans 80 ml d'HMPT et agite à température ambiante pendant 4 heures. On verse dans de l'eau, extrait avec de l'acétate d'éthyle, sèche et évapore sous pression réduite. On obtient 21,5 g de produit brut que l'on purifie par chromatographie en éluant avec le mélange benzène/acétate d'éthyle 9/1. On obtient 16,4 g de produit pur attendu.

### Stade C : Acide 23-carboxy-3,20-dioxo-16α-méthyl-21-norchola-1,4,9(11)-triène 24-oïque.

On mélange 16,4 g du diester préparé au stade précédent, 200 ml d'éthanol et 100 ml de soude 2N et agite 3 heures à température ambiante. Après évaporation de l'éthanol sous pression réduite, on dilue par addition d'un mélange glace + eau (1 litre) et acidifie avec de l'acide chlorhydrique. Après filtration, lavage et séchage on obtient 14,6 g du produit attendu. F = 170°C

### Stade D : Acide 3,20-dioxo-16α-méthyl-21-norchola-1,4,9(11)-triène 24-oïque.

On chauffe pendant 4 mn à l'aide d'un bain métallique préalablement chauffé à 180°C, le mélange constitué de 14,6 g du diacide obtenu au stade précédent dans 250 ml de HMPT, puis on verse dans 1500 ml d'un mélange glace + eau. Le monoacide qui précipite est filtré, lavé puis séché. On obtient 11,9 g du produit attendu. F = 208°C

### Stade E : 3,20-dioxo-16α-méthyl-21-norchola-1,4,9(11)-triène 24-oate de méthyle

A une solution de 11,9 g du monoacide formé au stade précédent dans 250 ml de dichlorométhane, on ajoute une solution de 13g/l de diazométhane dans le dichlorométhane puis évapore sous pression réduite. On obtient 12,3 g de produit attendu. F = 98°C

### Stade F : 9α-bromo-3,20-dioxo-11β-hydroxy-16α-méthyl-21-norchola-1,4-diène 24-oate de méthyle

A un mélange sous atmosphère inerte de 10,8 g d'ester méthylique dans 150 ml d'acétone on ajoute 7,3 g de N-bromo succinimide puis, à 0-10°C, 7,3 ml d'acide perchlorique et 35 ml d'eau. On mélange 2 heures à 0-5°C, puis verse dans 500 ml d'eau, filtre et sèche le précipité. On obtient 12,8 g du produit attendu. F = 230°C

### Stade G : 3,20-dioxo-11β-hydroxy-16α-méthyl-21-norchola-1,4-diène 24-oate de méthyle.

On ajoute 18 g d'acétate chromeux à un mélange sous atmosphère inerte de 12,8 g de bromhydrine préparée au stade précédent dans 50 ml de diméthylsulfoxyde (DMSO) et 6 ml de thiophénol, puis agite pendant 1 heure à température ambiante. On verse ensuite le mélange réactionnel dans 1 litre d'eau, extrait à l'acétate d'éthyle, lave et sèche la phase organique puis évapore sous pression réduite pour obtenir 16 g du produit brut attendu que l'on purifie par chromatographie en éluant avec le mélange benzène/acétate d'éthyle 7/3 puis par recristallisation dans l'éthanol à chaud. On obtient ainsi 3,4 g de produit pur attendu. F = 166°C
Infrarouge (CHCl₃)
- C=O: 1735 cm⁻¹ (ester), 1709 cm⁻¹ (20-ceto)
- C=C: 1609 cm⁻¹, 1626 cm⁻¹, 1664cm⁻¹
- OH: 3615 cm⁻¹

### EXEMPLE 6 : Exemple de Composition pharmaceutique renfermant un composé de formule (I)

On a préparé des comprimés à 50 mg de produit de l'exemple 1 comme principe actif.
Produit de l'exemple 1 50 mg
Excipient (talc, amidon, stéarate de magnésium)

### ETUDE PHARMACOLOGIQUE DES PRODUITS DE L'INVENTION

### I) Etude de la régulation de la transcription dans les cellules Hela transfectées.

Les cellules HeLa (réf ATCC : CCL-2) sont étalées en plaque de 6 puits, dans un milieu nutritif à la densité de 4.10⁵ cellules/ml, 24 heures avant la transfection et incubées à 37°C. Les transfections transitoires sont réalisées selon la méthode de précipité au phosphate de calcium. Dans le cas de l'étude de la transactivation les cellules sont transfectées avec 1 µg de plasmide GRE-tk-CAT, 1 µg de plasmide polyII β-GAL et le plasmide KS (Stratagène) q.s.p. 5 µg. Dans le cas de l'étude de la transrépression les cellules sont transfectées avec 3 µg du plasmide Coll(-517/+63)CAT, 250 ng de plasmide pSV-cjun, 1 µg de plasmide polyII β-GAL et le plasmide KS (Stratagène) q.s.p. 5 µg. Le précipité est laissé 16 heures en contact les cellules. Celles-ci sont ensuite rincées et recouvertes de milieu nutritif contenant différentes concentrations de Dexaméthasone ou de produits à étudier (10⁻⁹M, 10⁻⁸M, 10⁻⁷M, 10⁻⁶M). 24 heures après l'addition des produits, les cellules sont lysées dans 250 µl de tampon MOPS-NaCl-Triton X-100. La CAT est dosée dans les extraits cellulaires en utilisant le Kit ELISA (Boehringer Mannheim).

Les glucocorticoides classiques comme la Dexaméthasone provoquent une activation de la transcription du promoteur GRE-tk, et une inhibition de la transcription du promoteur de la collagénase.

| | Concentration (M) | Activation du GRE-tk (%) | Inhibition du promoteur Coll (%) |
|---|---|---|---|
| Dexaméthasone | 10-9 | 7,34+/-2,57 | 42,16+/-5,46 |
| | 10⁻⁸ | 53,38+/-2,65 | 87,60+/-2,94 |
| | 10⁻⁷ | 89,48+/-2,26 | 96,63+/-1,78 |
| | 10⁻⁶ | 98,82+/-1,18 | 93,08+/-2,87 |
| (Exemple 1) | 10⁻⁹ | 3,78+/-0,87 | 3,57+/-3,09 |
| | 10⁻⁸ | 27,74+/-1,05 | 37,12+/-6,7 |
| | 10⁻⁷ | 35,21+/-2,24 | 57,61+/-2,46 |
| | 10⁻⁶ | 37,56+/-1,9 | 81,79+/-2,98 |
| (Exemple 2) | 10⁻⁹ | 0 | 20,61+/-3,19 |
| | 10⁻⁸ | 1,34+/-1,33 | 36,2+/-1,39 |
| | 10⁻⁷ | 10,45+/-4,33 | 81,32+/-1,82 |
| | 10⁻⁶ | 16,92+/-3,55 | 75,57+/-1,34 |

### Conclusion :

Les produits des exemples 1 et 2 sont de bons inhibiteurs de la collagénase, mais sont de faibles activateurs de la transcription.

### 2) Activité anti-inflammatoire

### Test du granulome - Activité thymolytique

L'activité anti-inflammatoire a été recherchée selon le test classique du granulome. La technique utilisée est une modification de la méthode de Meier et Coll. (Experentia, 1950, 6, 469). Des rats femelles Wistar, pesant de 90 à 100 g, reçoivent une implantation de deux pellets de coton de 10 mg chacun sous la peau du thorax. Les produits sont aussitôt administrés par voie orale, à raison d'une fois par jour pendant quatre jours. Les animaux sont ensuite sacrifiés. Les pellets entourés de tissu de granulome formé, sont pesés à l'état frais, puis après séjour de dix-huit heures à 60°C. Le poids du granulome est obtenu par déduction du poids initial du coton.

Les thymus sont également prélevés et pesés afin de déterminer l'activité thymolytique des produits.

| Produit de l'exemple | Granulome ED 50(mg/kg/po) | Thymolyse ED 50(mg/kg/po) |
|---|---|---|
| Dexaméthasone | 0,1 | <0,05 |
| Prednisolone | 2,5 | 1,6 |
| EXEMPLE 1 | 5 | 2,5 |
| EXEMPLE 2 | >7 | 2,5 |

Conclusion : Les produits des exemples 1 et 2 présentent une activité anti-inflammatoire voisine de celle de la prednisolone.

### Test de l'oedème de l'oreille induit à l'huile de croton

Les produits ont également été testés dans le modèle de l'oedème de l'oreille de souris à l'huile de croton selon la méthode décrite dans Tonelli et coll. (Endocrinology, 1965, 77, 625-634). Un oedème de l'oreille est induit chez les souris mâles pesant 18 à 22 g, par application d'huile de croton (2 % v/v) dans une solution pyridine-eau-éther 4:1:14,6 (en volume). Les animaux sont sacrifiés 6 heures plus tard, les oreilles sont prélevées et pesées. La différence de poids entre l'oreille traitée à l'huile de croton et l'oreille controlatérale (non traitée) permet de déterminer le 100 % d'oedème. Les produits à tester sont dissous dans la solution d'huile de croton et appliqués sur l'oreille.

| Produit de l'exemple | Oedème à l'huile de croton (souris) ED 50 (µg/oreille) |
|---|---|
| Dexaméthasone | 1 |
| Prednisolone | 4 |
| EXEMPLE 1 | 10 |
| EXEMPLE 2 | 2 |

Conclusion : Le produit de l'exemple 2 présente dans ce test une activité anti-inflammatoire comprise entre celle de la prednisolone et de la dexaméthasone.

### 3) Activité immunosuppressive.

L'activité immunosuppresive a été recherchée selon le test d'hypersensibilité retardée décrit dans l'article de Hambleton et Coll. (Agents et Actions, 990, 29, 328). Des rats mâles Wistar pesant de 150 à 160 g sont sensibilisés au jour 0 avec une suspension de Mycobacterium Tuberculosis à 4 mg/ml dans de l'huile de paraffine par une injection sous cutanée à la base de la queue (0,1 ml/animal).

Les produits sont administrés par voie orale du jour 4 au jour 7.

Le jour 7, une heure après la dernière administration, les animaux reçoivent une injection de la fraction soluble de l'antigène déclenchant la réqction d'hypersensibilité : 0,4 mg/rat dans 0,2 ml en intraplantaire dans une patte arrière, l'autre reçoit la même quantité de solvant.

La mesure de l'oedème est pratiquée 24 heures après à l'aide d'un pléthysmomètre UGO BASIL de chez APELEX.

L'activité des produits est évaluée par le pourcentage de diminution de l'augmentation de la patte injectée par rapport à celle des animaux témoins.

| Produit de l'exemple | Hypersensibilité retardée (M.Tuberculosis, rat) ED 50 (mg/kg) |
|---|---|
| Dexaméthasone | 0,05 |
| Prednisolone | 5-20 |
| EXEMPLE 1 | 2,3 |
| EXEMPLE 2 | 1,5 |

Conclusion : La prednisolone présente une ED 50 en plateau entre 5 et 20 mg/kg, avec une inhibition maximale de 50%, les produits de l'exemple 1 et 2 ont des ED 50 de 2,3 et 1,5 mg/kg respectivement. Leur activité est dose-dépendente, avec une inhibition maximale de 82% (exemple 1) et 90% (exemple 2) à 10 mg/kg.

## Revendications

1. Produits de formule générale (I) : 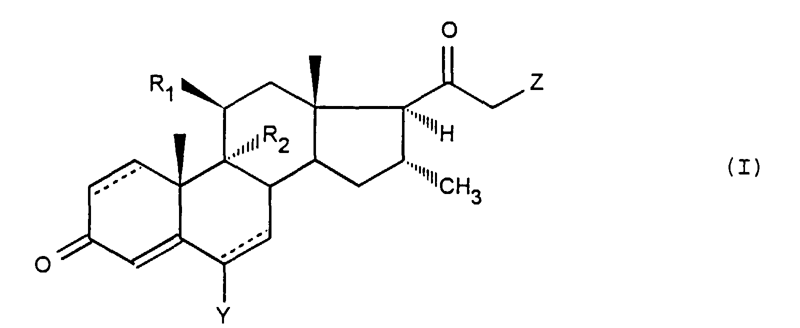 dans laquelle soit R₁ représente un atome d'halogène, un radical hydroxyle, un radical alkyloxy renfermant de 1 à 8 atomes de carbone, un radical acyloxy renfermant de 1 à 12 atomes de carbone, et R₂ représente un atome d'halogène ou un atome d'hydrogène, soit R₁ et R₂ forment ensemble une seconde liaison, Z est choisi parmi les groupements alkoxy renfermant de 1 à 8 atomes de carbone, alkylthio renfermant de 1 à 8 atomes de carbone non substitué ou substitué, arylthio carbocyclique renfermant de 6 à 12 atomes de carbone non substitué ou substitué, halogène, cyano, mercapto, thiocyanato, CO₂A et CH₂CO₂A, A étant un atome d'hydrogène ou un groupement alkyle renfermant de 1 à 8 atomes de carbone, Y représente un atome d'hydrogène ou un méthyle, le trait en pointillée en position 1-2 ou 5-6 représentant éventuellement une seconde liaison, ainsi que leurs sels d'addition avec les acides ou les bases, étant entendu que le 9α-fluoro-11β-hydroxy-16α-méthyl-21-chloro pregna-1,4-dièn-3,20-dione est exclu et étant entendu que lorsque R₁ et R₂ forment ensemble une double liaison, Z n'est pas un atome d'halogène.

2. Produits de formule (I) selon la revendication 1 dans laquelle R₁ est un radical hydroxyle et R₂ est un atome de fluor ainsi que leurs sels d'addition avec les acides ou les bases.

3. Produits de formule (I) selon la revendication 1 ou 2 dans laquelle le cycle B est saturé et Y est un atome d'hydrogène, ainsi que leurs sels d'addition avec les acides ou les bases.

4. Produits de formule (I) selon l'une quelconque des revendications 1 à 3 dans laquelle Z est choisi parmi les groupements cyano et alkylthio renfermant de 1 à 8 atomes de carbone.

5. Produits de formule (I) selon la revendication 1 dont les noms suivent :
- 9α-fluoro-11β-hydroxy-16α-méthyl-pregna-1,4-diene-3,20-dioxo-21-carbonitrile
- 9α-fluoro-11β-hydroxy-16α-méthyl-pregn-4-ène-3,20-dioxo-21-carbonitrile
- 9α-fluoro-11β-hydroxy-16α-méthyl-21-thiométhyl-pregna-1,4-diene-3,20-dione
- 3,20-dioxo-11β-hydroxy-16α-méthyl-21-nor cholane 1,4-diène-24-oate de méthyle
- 21-fluoro-11β-hydroxy-16α-méthyl-pregna-1,4-diène-3,20-dione
- 21-thio-(2-hydroxyéthylène)-9α,11β-dichloro-16α-méthylpregna-1,4-diene-3,20-dione
- 21-thio- (2-acétyloxyéthylène)-9α,11β-dichloro-16α-méthylpregna-1,4-diene-3,20-dione
- 9α,11β-dichloro-21-fluoro-16α-méthyl-pregna-1,4-diene-3,20-dione.

6. Procédé de fabrication des produits de formule (I) tels que définis à la revendication 1 caractérisé en ce que l'on soumet un produit de formule générale (II) : 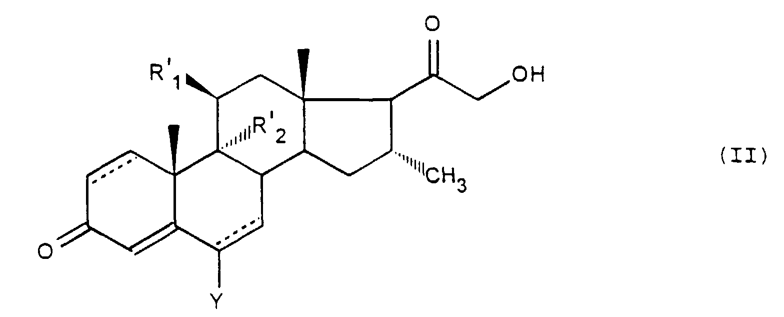 dans laquelle soit R'₁ est un hydroxyle et R'₂ est un halogène ou un hydrogène, soit R'₁ est un halogène et R'₂ est un halogène ou un hydrogène, soit R'₁ et R'₂ forment ensemble une seconde liaison et Y est tel que défini précédemment, à l'action d'un réactif d'activation de l'alcool de formule Hal-SO₂-B, Hal étant un atome de brome ou de chlore et B étant un radical alkyle renfermant de 1 à 5 atomes de carbone, non substitué ou substitué par 1 à 5 atomes d'halogène, ou un radical phényle ou naphtyle non substitué ou substitué par 1 à 5 groupement alkyle renfermant de 1 à 6 atomes de carbone, afin d'obtenir un composé de formule générale (III) : 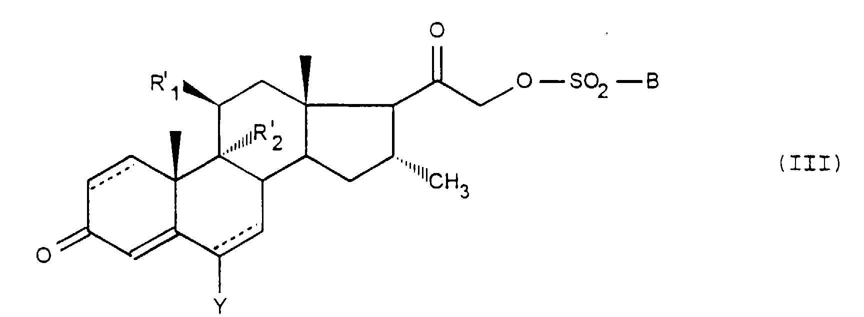 dans laquelle B est tel que défini précédemment, produits de formule (III) que l'on soumet à l'une ou plusieurs des réactions suivantes dans un ordre approprié, afin d'obtenir un produit de formule générale (I) :
- action d'un réactif de chloration, de iodation, de bromation ou de fluoration
- action d'un alkylthiol ou d'un arylthiol éventuellement substitué
- action d'une thioamide ou d'une thiourée suivie d'une hydrolyse
- action de KCN
- hydrolyse du groupement cyano en milieu acide puis éventuellement estérification ou salification,
- action successivement de CH₂(COOEt)₂, d'une réaction de saponification puis d'une réaction de décarboxylation suivie éventuellement d'une réaction d'estérification ou de salification
- action de KSCN ou NaSCN
- action d'un alcool ou d'un alcoolate
- réaction d'acylation en position 11
- réaction d'alkylation en position 11
- réduction de la double liaison en position 1-2
- formation de la double liaison en position 1-2
- réaction de déshydratation afin de former une double liaison en position 9-11
- salification.

7. A titre de médicaments, les composés de formule (I) tels que définis à la revendication 1, ainsi que leurs sels d'addition avec les acides ou les bases pharmaceutiquement acceptables.

8. A titre de médicaments, les composés de formule (I) tels que définis à l'une des revendications 2 à 4, ainsi que leurs sels d'addition avec les acides ou les bases pharmaceutiquement acceptables.

9. A titre de médicaments, les composés de formule (I) tels que définis à la revendication 5.

10. Compositions pharmaceutiques renfermant les médicaments tels que définis aux revendications 7 à 9.

11. A titre de produits industriels, les produits de formule générale (III) tels que définis à la revendication 6, étant entendu que les composés de formule (III) suivants sont exclus :
- 21-(méthanesulfonyloxy)-16α-méthylpregna-1,4,9(11)-trièn-3,20-dione
- 21-(méthanesulfonyloxy) 11β-hydroxy-16α-méthyl-pregna-1,4-dièn-3,20-dione
- 21(4-méthylphénylsulfonyloxy)-16α-méthyl-pregna-1,4,9(11)-trièn-3,20-dione.
- 9α-fluoro-21-(méthanesulfonyloxy)-11β-hydroxy-16α-méthyl-pregna-1,4-dièn-3,20-dione.

## Claims

1. Products of the general formula (I): 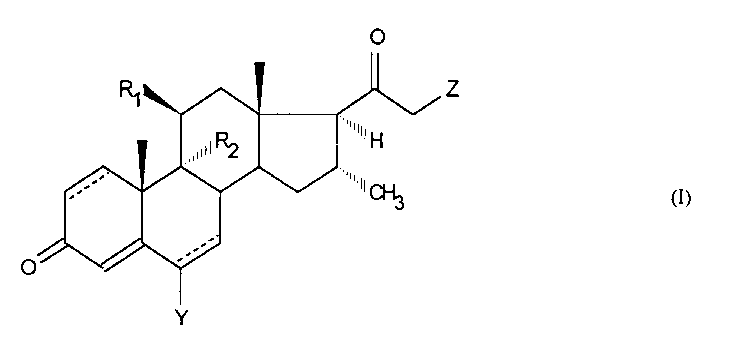 in which either R₁ represents a halogen atom, a hydroxyl radical, an alkyloxy radical with 1 to 8 carbon atoms, an acyloxy radical with 1 to 12 carbon atoms, and R₂ represents a halogen atom or a hydrogen atom, or R₁ and R₂ together form a second bond, Z is selected from the alkoxy groups with 1 to 8 carbon atoms, alkylthio groups with 1 to 8 carbon atoms whether substituted or not, carbocyclic arylthio groups with 6 to 12 carbon atoms whether substituted or not, halogen, cyano, mercapto, thiocyanato, CO₂A and CH₂CO₂A, groups where A is a hydrogen atom or an alkyl group with 1 to 8 carbon atoms, Y represents a hydrogen atom or a methyl, the dotted line in position 1-2 or 5-6 represents a possible second bond, and the addition salts of such compounds with acids or bases, provided that 9α-fluoro-11β-hydroxy-16 a-methyl-21-chloro-pregna-1,4-dien-3, 20-dione is excluded and also that when R₁ and R₂ together form a double bond, Z is not a halogen atom.

2. Products of formula (I) according to Claim 1 in which R₁ is a hydroxyl radical and R₂ is a fluorine atom, and their addition salts with acids or bases.

3. Products of formula (I) according to Claim 1 or 2 in which the B ring is saturated and Y is a hydrogen atom, and their addition salts with acids or bases.

4. Products of formula (I) according to any of Claims 1 to 3, in which Z is selected from cyano and alkylthio groups having from 1 to 8 carbon atoms.

5. Products of formula (I) according to Claim 1, whose names are as follows:
- 9α-fluoro-11β-hydroxy-16α-methyl-pregna-1,4-diene-3, 20-dioxo-21-carbonitrile
- 9α-fluoro-11β-hydroxy-16α-methyl-pregn-4-ene-3,20-dioxo-21-carbonitrile
- 9α-fluoro-11β-hydroxy-16α-methyl-21-thiomethyl-pregna-1,4-diene-3,20-dione
- methyl 3,20-dioxo-11β-hydroxy-16α-methyl-21-nor cholane-1,4-diene-24-oate
- 21-fluoro-11β-hydroxy-16α-methyl-pregna-1,4-diene-3, 20-dione
- 21-thio-(2-hydroxyethylene)-9α,11β-dichloro-16α-methyl-pregna-1,4-diene-3,20-dione
- 21-thio-(2-acetyloxyethylene)-9α,11β-dichloro-16α-methyl-pregna-1,4-diene-3,20-dione
- 9α, 11β-dichloro-21-fluoro-16α-methyl-pregna-1,4-diene-3, 20-dione.

6. Process for the production of products of formula (I) as defined in Claim 1, characterised in that a product with general formula (II): 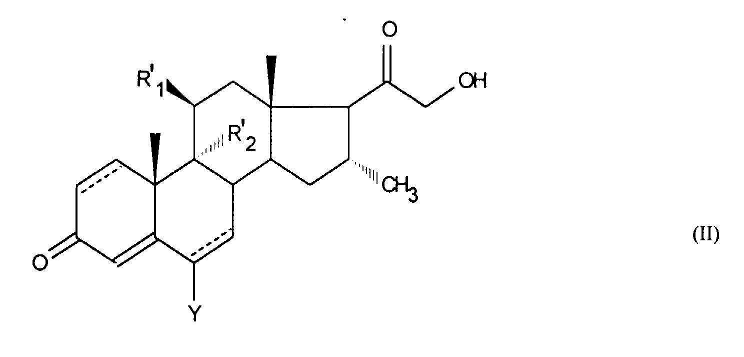 in which either R'₁ is a hydroxyl and R'₂ is a halogen or a hydrogen, or R'₁ is a halogen and R'₂ is a halogen or a hydrogen, or R'₁ and R'₂ together form a second bond and Y is as previously defined, is subjected to the action of an alcohol-activating reagent of formula Hal-SO₂-B, where Hal is a bromine or chlorine atom and B is an alkyl radical with 1 to 5 carbon atoms whether substituted by 1 to 5 halogen atoms, or a phenyl or naphthyl radical whether substituted by 1 to 5 alkyl groups containing 1 to 6 carbon atoms, in order to obtain a compound of general formula (III): 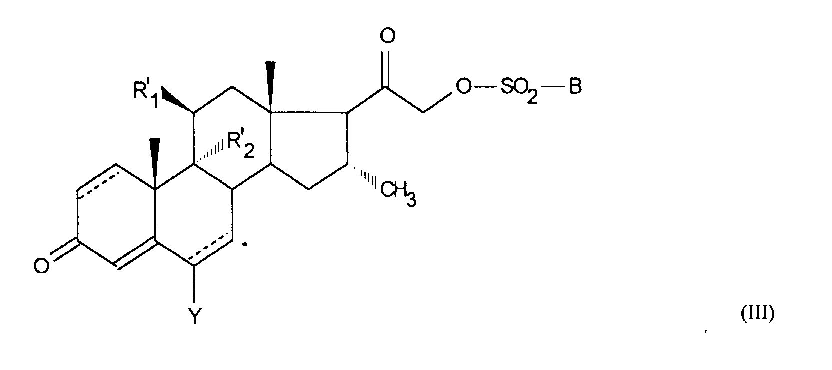 in which B is as previously defined,
which products of formula (III) are subjected to one or more of the following reactions in an appropriate order
- action of a chlorination, iodination, bromination or fluorination reagent,
- action of a possibly-substituted alkylthiol or arylthiol,
- action of a thioamide or thiourea followed by hydrolysis,
- action of KCN,
- hydrolysis of the cyano group in an acid medium and then possible esterification or salification,
- successive action of CH₂(COOEt)₂, a saponification reaction and then a decarboxylation reaction, if necessary followed by an esterification or salification reaction,
- action of KSCN or NaSCN,
- action of an alcohol or an alcoholate,
- acylation reaction at position 11,
- alkylation reaction at position 11,
- reduction of the double bond at position 1-2,
- formation of the double bond at position 1-2,
- dehydration reaction to form a double bond at position 9-11,
- salification.
- to obtain a product of the general formula (I).

7. As medicaments, the compounds of formula (I) as defined in Claim 1 and their addition salts with pharmaceutically acceptable acids or bases.

8. As medicaments, the compounds of formula (I) as defined in any of Claims 2 to 4, and their addition salts with pharmaceutically acceptable acids or bases.

9. As medicaments, the compounds of formula (I) as defined in Claim 5.

10. Pharmaceutical compositions containing the medicaments defined in Claims 7 to 9.

11. As industrial products, the products of general formula (III) as defined in Claim 6, it provided that the following compounds of formula (III) are excluded:
- 21-(methanesulphonyloxy)-16α-methyl-pregna-1,4,9 (11)-trien-3,20-dione,
- 21-(methanesulphonyloxy)-11β-hydroxy-16α-methyl-pregna-1,4-dien-3,20-dione,
- 21-(4-methylphenylsulphonyloxy)-16α-methyl-pregna-1,4,9 (11)-trien-3,20-dione,
- 9α-fluoro-21-(methanesulphonyloxy)-11β-hydroxy-16α-methyl-pregna-1,4-dien-3,20-dione.

## Patentansprüche

1. Verbindungen der allgemeinen Formel (I): 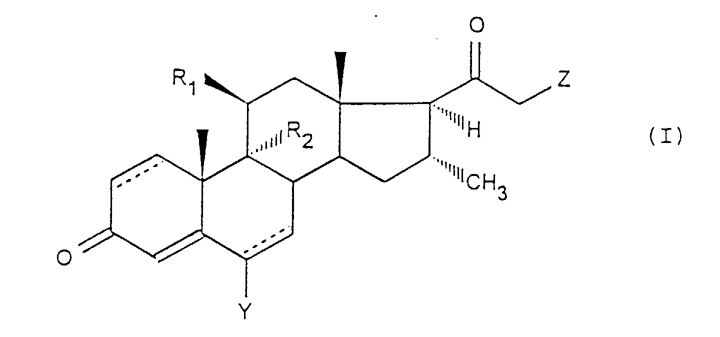 worin entweder R₁ ein Halogenatom, einen Hydroxylrest, einen Alkyloxyrest mit 1 bis 8 Kohlenstoffatomen, einen Acyloxyrest mit 1 bis 12 Kohlenstoffatomen bezeichnet, und R₂ ein Halogenatom oder ein Wasserstoffatom bezeichnet, oder R₁ und R₂ zusammen eine Doppelbindung bilden, Z aus den Resten Alkoxy mit 1 bis 8 Kohlenstoffatomen, Alkylthio mit 1 bis 8 Kohlenstoffatomen, die substituiert sind oder nicht, carbocyclisches Arylthio mit 6 bis 12 Kohlenstoffatomen, das substituiert ist oder nicht, Halogen, Cyano, Mercapto, Thiocyanato, CO₂A und CH₂CO₂A ausgewählt ist, wobei A ein Wasserstoffatom oder eine Alkylgruppe mit 1 bis 8 Kohlenstoffatomen ist, Y ein Wasserstoffatom oder Methyl bedeutet, die punktierte Linie in Position 1-2 oder 5-6 gegebenenfalls eine Doppelbindung bezeichnet, sowie ihre Additionssalze mit Säuren oder Basen, mit der Maßgabe, daß 9α-Fluor-11β-hydroxy-16α-methyl-21-chlorpregna-1,4-dien-3,20-dion ausgeschlossen ist und mit der Maßgabe, daß wenn R₁ und R₂ zusammen eine Doppelbindung bilden, Z kein Halogenatom ist.

2. Verbindungen der Formel (I) nach Anspruch 1, worin R₁ ein Hydroxylrest ist und R₂ ein Fluoratom ist, sowie ihre Additionssalze mit Säuren oder Basen.

3. Verbindungen der Formel (I) nach Anspruch 1 oder 2, worin der Ring B gesättigt ist und Y ein Wasserstoffatom ist, sowie ihre Additionssalze mit Säuren oder Basen.

4. Verbindungen der Formel (I) nach einem der Ansprüche 1 bis 3, worin Z aus den Gruppierungen Cyano und Alkylthio mit 1 bis 8 Kohlenstoffatomen ausgewählt ist.

5. Verbindungen der Formel (I) nach Anspruch 1, nämlich:
- 9α-Fluor-11β-hydroxy-16α-methylpregna-1,4-dien-3,20-dioxo-21-carbonitril,
- 9α-Fluor-11β-hydroxy-16α-methylpregn-4-en-3,20-dioxo-21-carbonitril,
- 9α-Fluor-11β-hydroxy-16α-methyl-21-thiomethylpreg-na-1,4-dien-3,20-dion,
- Methyl-3,20-dioxo-11β-hydroxy-16α-methyl-21-norcho-lan-1,4-dien-14-oat,
- 21-Fluor-11β-hydroxy-16α-methylpregna-1,4-dien-3,20-dion,
- 21-Thio-(2-hydroxyethylen)-9α,11β-dichlor-16α-methylpregna-1,4-dien-3,20-dion,
- 21-Thio-(2-acetyloxyethylen)-9α,11β-dichlor-16α-methylpregna-1,4-dien-3,20-dion,
- 9α,11β-Dichlor-21-fluor-16α-methylpregna-1,4-dien-3,20-dion.

6. Verfahren zur Herstellung von Verbindungen der Formel (I) wie in Anspruch 1 definiert, dadurch **gekennzeichnet**, daß man eine Verbindung der allgemeinen Formel (II): 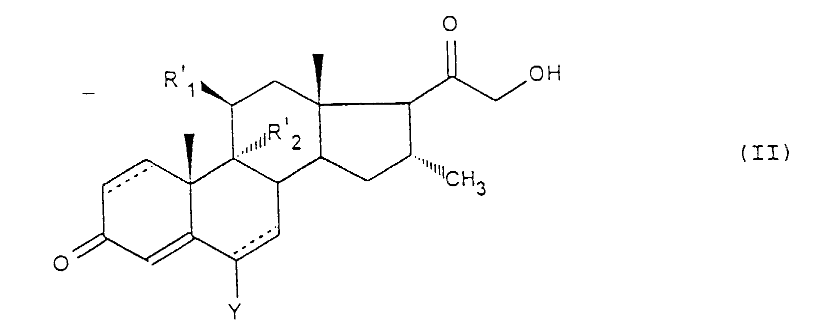 worin entweder R'₁ ein Hydroxyl ist und R'₂ ein Halogen oder ein Wasserstoff ist oder R'₁ ein Halogen ist und R'₂ ein Halogen oder ein Wasserstoff ist, oder R'₁ und R'₂ zusammen eine Doppelbindung bilden, und Y wie vorstehend definiert ist, der Wirkung eines Aktivierungsreagenses des Alkohols der Formel Hal-SO₂-B unterwirft, wobei Hal ein Brom- oder Chloratom ist und B ein Alkylrest mit 1 bis 5 Kohlenstoffatomen ist, der unsubstituiert oder durch 1 bis 5 Halogenatome substituiert ist oder ein Phenyl- oder Naphthylrest ist, der unsubstituiert ist oder durch 1 bis 5 Alkylreste mit 1 bis 6 Kohlenstoffatomen substituiert ist, um eine Verbindung der allgemeinen Formel (III) zu erhalten: 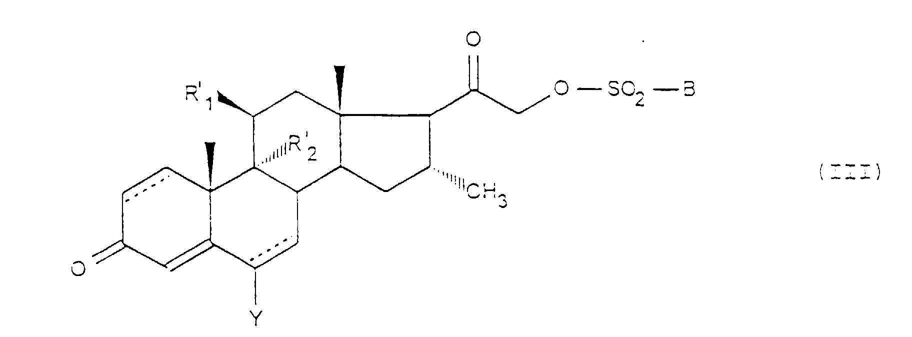 worin B wie vorstehend definiert ist, wobei man die Verbindung der Formel (III) einer oder mehreren der folgenden Reaktionen in einer geeigneten Reihenfolge unterwirft, um eine Verbindung der allgemeinen Formel (I) zu erhalten:
- Wirkung eines Chlorierungs-, Iodierungs-, Bromierungs- oder Fluorierungsreagenses,
- Wirkung eines Alkylthiols oder eines Arylthiols, das gegebenenfalls substituiert ist,
- Wirkung eines Thioamids oder eines Thioharnstoffs, gefolgt von einer Hydrolyse,
- Wirkung von KCN,
- Hydrolyse der Cyanogruppierung in saurem Medium, dann gegebenenfalls Veresterung oder Salzbildung,
- sukzessive Wirkung von CH₂(COOEt)₂, einer Verseifungsreaktion, dann einer Decarboxylierungsreaktion, gegebenenfalls gefolgt von einer Veresterungs- oder Salzbildungsreaktion,
- Wirkung von KSCN oder NaSCN,
- Wirkung eines Alkohols oder eines Alcoholats,
- Acylierungsreaktion in Position 11,
- Alkylierungsreaktion in Position 11,
- Reduktion der Doppelbindung in Position 1-2,
- Bildung der Doppelbindung in Position 1-2,
- Dehydratisierungsreaktion, um eine Doppelbindung in Position 9-11 zu bilden,
- Salzbildung.

7. Verbindungen der Formel (I), wie in Anspruch 1 definiert, sowie ihre Additionssalze mit pharmazeutisch verträglichen Säuren oder Basen als Medikamente.

8. Verbindungen der Formel (I), wie in einem der Ansprüche 2 bis 4 definiert, sowie ihre Additionssalze mit pharmazeutisch verträglichen Säuren oder Basen als Medikamente.

9. Verbindungen der Formel (I), wie in Anspruch 5 definiert, als Medikamente.

10. Pharmazeutische Zusammensetzungen, umfassend die Medikamente wie in den Ansprüchen 7 bis 9 definiert.

11. Verbindungen der allgemeinen Formel (III), wie in Anspruch 6 definiert, als Industrieprodukte mit der Maßgabe, daß die folgenden Verbindungen der Formel (III) ausgeschlossen sind:
- 21-(Methansulfonyloxy)-16α-methylpregna-1,4,9(11)-trien-3,20-dion,
- 21-(Methansulfonyloxy)-11β-hydroxy-16α-methyl-pregna-1,4-dien-3,20-dion
- 21-(4-Methylphenylsulfonyloxy)-16α-methylpregna-1,4,9(11)-trien-3,20-dion
- 9α-Fluor-21-(methansulfonyloxy)-11β-hydroxy-16α-methylpregna-1,4-dien-3,20-dion.
